Europäisches Patentamt

European Patent Office

Office européen des brevets

(19)

(11) **EP 1 715 323 A1**

(12) **EUROPEAN PATENT APPLICATION**

(43) Date of publication:
**25.10.2006 Bulletin 2006/43**

(51) Int Cl.:
*G01N 15/02* (2006.01)    *G01N 33/28* (2006.01)

(21) Application number: **05254926.8**

(22) Date of filing: **05.08.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **BP OIL INTERNATIONAL LIMITED**
**Sunbury-on-Thames,**
**Middlesex TW16 7BP (GB)**

(72) Inventors:
• **Clark, Alisdair Quentin**
**Reading,**
**Berkshire, RG8 7QR (GB)**

• **Crighton, James S.**
**Reading,**
**Berkshire, RG8 7QR (GB)**

(74) Representative: **Perkins, Nicholas David**
**BP International Limited**
**Patents and Agreements Division,**
**Chertsey Road**
**Sunbury-on-Thames,**
**Middlesex TW16 7LN (GB)**

(54) **Method for determining the concentration and/or size distribution of solid particles in middle distillate fuels**

(57)    A method for determining the concentration and/or particle size distribution of suspended solid particles in a middle distillate fuel, which middle distillate fuel comprises suspended solid particles and suspended undissolved water, and which method comprises the steps of

(i) solubilising the suspended water to produce a solution of water in the fuel and
(ii) measuring the concentration and/or particle size distribution of suspended solid particles in the solution produced in step (i) by an optical analysis technique.

EP 1 715 323 A1

**Description**

This invention relates to an analytical method, and in particular to an analytical method for determining the concentration and/or particle size distribution of suspended solid particles in middle distillate fuels.

Fuel performance is influenced, amongst other things, by the nature and the concentration of impurities contained therein. Characteristics of a fuel that are often considered desirable include a low water content, and a visual appearance that is clear and free from matter in suspension. Matter which may be in suspension in fuel includes suspended solid particles, for example dirt, pipe scale, sand, rust, microbes and their waste, and suspended undissolved water. The presence of water, including suspended undissolved water, may reduce the calorific value of the fuel, and may also cause blockage of fuel lines by formation of ice. The presence of suspended solid particles may also block fuel lines and filters, and cause wear by abrasion.

Typically, a visual test is carried out to determine whether suspended matter is present in unacceptably high concentrations in a fuel. Such a test is exemplified by ASTM D-4176. A problem with such a test is that it is not quantitative, the result being given either as a "pass" or a "fail". The concentration of suspended matter is not measured. Another problem is that such a test cannot discriminate between suspended matter that is related to solid particles, and suspended matter related to suspended undissolved water. A further problem is that wearing of fuel lines can be caused by particles of suspended solid matter that are significantly smaller than can be detected by the human eye. Yet another problem is that the human eye may not be able to detect suspended undissolved water at concentrations that can cause problems in aircraft engines.

The use of gravimetric methods to measure the mass of suspended solid particles has been previously described, for example in test methods IP423 and ASTM D-5452. Water content may be measured using known techniques, such as Karl-Fischer titration as described, for example, in test methods IP438 and IP439. However, these tests need to be performed separately using different apparatus, and often the analysis may take some time to complete. Furthermore, gravimetric methods are prone to suffer from poor reliability.

There remains a need for a method for determining the concentration and/or particle size distribution of suspended solid particles in a distillate fuel that mitigates or at least reduces such problems.

Thus, according to the present invention, there is provided a method for determining the concentration and/or particle size distribution of suspended solid particles in a middle distillate fuel, which middle distillate fuel comprises suspended solid particles and suspended undissolved water, and which method comprises the steps of

(i) solubilising the suspended water to produce a solution of water in the fuel and
(ii) measuring the concentration and/or particle size distribution of suspended solid particles in the solution produced in step (i) by an optical analysis technique.

The present invention overcomes the technical problem identified above by solubilising any suspended undissolved water prior to measuring the concentration and/or particle size distribution of suspended solid particles. An advantage of the present invention is that the concentration and/or particle size distribution of suspended solid particles may be determined independently of the concentration and/or particle size distribution of suspended undissolved water.

Optical analysis techniques for measuring the concentration and/or particle size distribution of particles of suspended matter may also be used to determine the concentration and/or particle size distribution of suspended solid particles and undissolved water in the middle distillate fuel before the water solubilising agent is added.

In a further aspect, the present invention also provides a method for determining the concentration and/or particle size distribution of suspended solid particles and the concentration of suspended undissolved water in a middle distillate fuel.

Thus, according to a second embodiment of the present invention, prior to solubilising the water in step (i), the concentration and/or particle size distribution of suspended solid particles and suspended undissolved water is measured by an optical analysis technique and the concentration and/or particle size distribution of suspended undissolved water in the middle distillate fuel is determined by subtracting the concentration and/or particle size distribution of suspended solid particles measured in step (ii) from the concentration and/or particle size distribution of suspended solid particles and undissolved water measured prior to solubilising the water.

An advantage of the present invention is that the method is relatively quick compared, for example, to titrimetric or gravimetric methods. An advantage of the second aspect of the present invention in particular is that the concentration and/or particle size distribution of suspended undissolved water and suspended solid particles in the middle distillate fuel may both be determined quantitatively. A further advantage is that the same apparatus may be used.

The suspended undissolved water may be solubilised by adding a water solubilising agent to the fuel to dissolve the suspended water and produce a solution of the water in the fuel and/or by heating the fuel to a temperature sufficiently elevated to dissolve the suspended water and produce a solution of water in the fuel.

The water solubilising agent used in the methods of the present invention is any suitable compound that is soluble in the middle distillate fuel, and which will also cause dissolution of any suspended undissolved water in the middle distillate fuel. Suitable classes of compounds would include alcohols, glycol ethers, ketones, esters, aldehydes and carboxylic acids. Suitable alcohols include isopropyl alcohol, tertiary-butyl alcohol, and ethanol; suitable glycol ethers include diethylene glycol monomethyl ether; suitable ketones include acetone; suitable esters include methyl formate; suitable aldehydes include propanal and 2-methyl propanal; and suitable carboxylic acids include 2-methyl propanoic acid. The water solubilising agent may be a mixture of more than one compound. The water solubilising agent, and its mixture with the middle distillate fuel, desirably has a flash point of greater than 38°C.

The volume of solubilising agent added to the middle distillate fuel is sufficient to ensure dissolution of the undissolved suspended water, although preferably the added volume is minimised so that the concentration of any particulates in the fuel is not excessively diluted. To compensate for dilution of the fuel by the added solubilising agent, the concentration of particles in the fuel may suitably be calculated according to the following equation:

$$V_{pcorr} = V_p \ x \ (V_{mdf} + V_{sa})/V_{mdf}$$

where $V_{mdf}$ is the volume of middle distillate fuel, $V_{sa}$ is the volume of added solubilising agent, $V_p$ is the volume of particles measured, and $V_{pcorr}$ is the corrected particle volume to account for the increase in volume after adding the solubilising agent.

The temperature to which the middle distillate fuel is heated in order to solubilise the undissolved suspended water will vary depending on the chemical composition of the fuel and the ambient temperature of the fuel before heating. However, typically the distillate fuel is heated to a temperature between the solubility point of the undissolved suspended water and below that at which the jet fuel will begin to boil, for example in the range from 0°C to 140°C, for example from 5°C to 100°C or from 20°C to 100°C, and preferably in the range from 35°C to 90°C.

The concentration and/or particle size distribution, of suspended matter (suspended undissolved water and/or suspended solid particles) is measured by an optical analysis technique. Suitable techniques which may be used in the methods of the present invention include light extinction, light scattering, or interferometry. Preferably, the optical analysis technique is light extinction.

Determining the concentration and/or particle size distribution, of suspended matter by light extinction involves irradiating the fuel with light of at least one wavelength, preferably laser light produced for example by a diode laser, and measuring the obscuration of the light by a detector which is sensitive to the intensity of light transmitted by the fuel, for example by means of a photodiode. The laser light wavelength or wavelengths are preferably in the visible or infrared region of the electromagnetic spectrum. Preferably, the light is of a wavelength which is strongly absorbed by water. The light that is either blocked, or whose intensity is reduced by particles of suspended matter in the fuel, is proportional to the concentration and size of the particles of suspended matter. Preferably, the concentration distribution determination is achieved using a laser particle counter, such as a UCC-Parker LCM-20 Particle Counter, which can not only give the total number of particles counted in a sample of middle distillate fuel within a specified period of time; but is also able to determine a particle size distribution, which can be correlated to the particle concentration.

Concentrations of particles of suspended matter (suspended solid particles and/or suspended undissolved water) may be expressed in various forms, for example as a volume. ratio (v/v), as a weight ratio (w/w), as a weight to volume ratio (w/v), as a number of particles per unit volume of fuel, or as a number of particles counted per unit time.

A further advantage of the present invention is the possibility of simultaneously obtaining particle size distributions.

Particle diameters that may be measured by optical analysis techniques, in particular light extinction, are suitably up to 1mm, preferably up to 100 microns (micrometers), for example from 2 to 100 microns. Particle size distributions may be expressed in particle diameter ranges of from 2 to 200 microns. As an example, a typical particle size distribution may be expressed as number of particles with diameters between 2 and 5 microns, between greater than 5 and 15 microns, between greater than 15 and 25 microns, between greater than 25 microns and 50 microns, and greater than 50 microns. Particle size distributions may be expressed in terms of a frequency or quantity of particles within a stipulated series of size ranges.

The middle distillate fuel may be any fuel that is generally denser than gasoline, for example kerosene, jet fuel or diesel. It may originate as a direct fraction from the crude distillation unit of a typical refinery, or may be produced by suitably treating heavier crude oil fractions, such as atmospheric gas oil or vacuum gas oil, by suitable means such as hydrocracking or fluid catalytic cracking. The methods of the present invention are suitable for either desulphurised or non-desulphurised fuel. Preferably, the middle distillate fuel is jet fuel. The methods may also be applied to heavier oils, such as lubrication oils, transmission fluids or heavy fuel oils, optionally in the presence of a viscosity reducing solvent, for example a hydrocarbon-based solvent such as white spirit or mixed xylene.

The methods of the present invention may be performed on a sample of middle distillate fuel taken, for example, from a storage tank and analysed ex situ, for example in a laboratory. Alternatively, the methods may be used in an on-line configuration, wherein a sample is extracted from a reservoir of middle distillate fuel, such as a storage tank, wherein the concentration and/or particle size distribution of suspended matter (and optionally particles size distribution) both before and after solubilising the suspended undissolved water is measured using an analysis chamber. The source of laser light may be remote from the analysis chamber, the light being transmitted by means of optical fibres. Similarly, the light emitted from the analysis chamber, after interaction with the middle distillate fuel sample, may also be transmitted to the detector by means of optical fibres. The analysed middle distillate fuel may then either be transferred back to the fuel reservoir, or sent to waste.

For the methods of the present invention in which the fuel is heated to solubilse the suspended undissolved water, the methods may also be performed either remotely from a middle distillate fuel reservoir, such as in a laboratory, or in an on-line configuration located at or close to the middle distillate fuel reservoir or source.

These methods may be performed using an apparatus with a single analysis chamber that has a heater for heating the middle distillate fuel to dissolve the suspended undissolved water. When a single analysis chamber is used to measure suspended solid particles and suspended undissolved water at a first temperature and suspended solid particles at an elevated temperature, an appropriate correction or calibration may be required in order to correct for any variations in concentration measurements using the optical analysis technique at two different temperatures.

The methods of the present invention may be performed using two separate analysis chambers connected in series, in which one is used for determination of the concentration and/or particle size distribution of suspended solid particles and suspended undissolved water at a temperature below the solubility point of the suspended undissolved water, and the other is used for determining the concentration of suspended solid particles at a temperature at or above which the suspended undissolved water dissolves in the middle distillate fuel.

**Claims**

1. A method for determining the concentration and/or particle size distribution of suspended solid particles in a middle distillate fuel, which middle distillate fuel comprises suspended solid particles and suspended undissolved water, and which method comprises the steps of

   (i) solubilising the suspended water to produce a solution of water in the fuel and
   (ii) measuring the concentration and/or particle size distribution of suspended solid particles in the solution produced in step (i) by an optical analysis technique.

2. A method as claimed in claim 1 in wherein prior to solubilising the water in step (i), the concentration and/or particle size distribution of suspended solid particles and suspended undissolved water is measured by an optical analysis technique and the concentration and/or particle size distribution of suspended undissolved water in the middle distillate fuel is determined by subtracting the concentration and/or particle size distribution of suspended solid particles measured in step (ii) from the concentration and/or particle size distribution of suspended solid particles and undissolved water measured prior to solubilising the water.

3. A method as claimed in claim 1 or claim 2 in which the suspended undissolved water is solubilised by adding a water solubilising agent to the fuel to dissolve the suspended water and produce a solution of the water in the fuel and/or by heating the fuel to a temperature sufficiently elevated to dissolve the suspended water and produce a solution of water in the fuel.

4. A method as claimed in claim 3 wherein the water solubilising agent is selected from the group consisting of alcohols, glycol ethers, ketones, esters, aldehydes and carboxylic acids.

5. A method as claimed in claim 4 in which the water solubilising agent is a compound selected from the group consisting of isopropyl alcohol, tertiary-butyl alcohol, ethanol, diethylene glycol monomethyl ether, acetone, methyl formate, propanal, 2-methyl propanal and 2-methyl propanoic acid.

6. A method as claimed in claim 4 or claim 5 in which the water solubilising agent is a mixture of more than one compound.

7. A method as claimed in any one of claims 3 to 6 in which the suspended undissolved water is solubilised by heating the fuel to a temperature of up to 140°C.

8. A method as claimed in claim 7 in which the suspended undissolved water is solubilised by heating the fuel to a temperature of from 5 to 100°C.

9. A method as claimed in claim 8 in which the suspended undissolved water is solubilised by heating the fuel to a temperature in the range from 35 to 90°C.

10. A method as claimed in any one of claims 1 to 9 in which the middle distillate fuel is kerosene, jet fuel or diesel.

**European Patent Office**

**EUROPEAN SEARCH REPORT**

Application Number

EP 05 25 4926

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 4 024 400 A (BLYTAS ET AL) 17 May 1977 (1977-05-17) | 1,7-10 | G01N15/02 G01N33/28 |
| Y | * the whole document * | 1-10 | |
| Y | US 6 064 480 A (MOUNTAIN ET AL) 16 May 2000 (2000-05-16) * the whole document * | 1-10 | |
| A | US 3 449 567 A (JAMES PETER OLIVIER ET AL) 10 June 1969 (1969-06-10) * the whole document * | 1 | |

TECHNICAL FIELDS SEARCHED (Int.Cl.7)

G01N

The present search report has been drawn up for all claims

| Place of search | Date of completion of the search | Examiner |
|---|---|---|
| Munich | 27 September 2005 | Bravin, M |

**ANNEX TO THE EUROPEAN SEARCH REPORT
ON EUROPEAN PATENT APPLICATION NO.**

EP 05 25 4926

This annex lists the patent family members relating to the patent documents cited in the above-mentioned European search report.
The members are as contained in the European Patent Office EDP file on
The European Patent Office is in no way liable for these particulars which are merely given for the purpose of information.

27-09-2005

| Patent document cited in search report | | Publication date | Patent family member(s) | Publication date |
|---|---|---|---|---|
| US 4024400 | A | 17-05-1977 | NONE | |
| US 6064480 | A | 16-05-2000 | NONE | |
| US 3449567 | A | 10-06-1969 | NONE | |

EPO FORM P0459

For more details about this annex : see Official Journal of the European Patent Office, No. 12/82